# EUROPÄISCHE PATENTANMELDUNG

(11) **EP 0 829 522 A1**
(43) Veröffentlichungstag der Anmeldung: **18.03.1998**
(21) Anmeldenummer: 97114980.2
(22) Anmeldetag: 29.08.1997
(51) Int. Cl.: C09B 25/00, C08K 5/3437

(54) **Verfahren zur Herstellung von Chinophthalonen**

(30) Priorität: 11.09.1996 DE 19636880
(71) Anmelder: BAYER AG, 51368 Leverkusen (DE)
(72) Erfinder: Kalz, Dietmar, Dr., 53819 Neunkirchen (DE); Michaelis, Stephan, Dr., 51519 Odenthal (DE); Reinhardt, Karl-Heinz, 40789 Monheim (DE)

(57) **Zusammenfassung**

Verfahren zur Herstellung von Chinophthalonen der Formel (I) 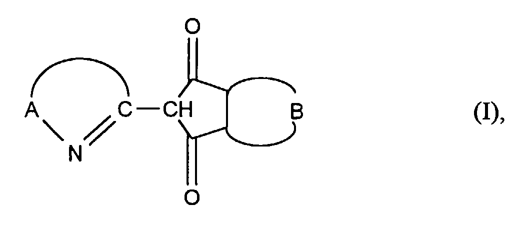 worin die Gruppierung A einen unsubstituierten oder substituierten heteroaromatischen Ring vervollständigt und die Gruppierung B einen aromatischen Ring vervollständigt, der unsubstituiert oder substituiert ist, durch Umsetzung einer heterocyclischen Verbindung der allgemeinen Formel (II) 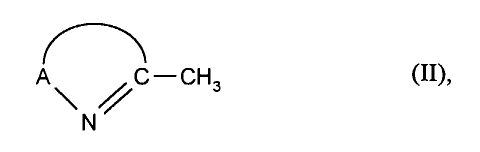 mit einer aromatischen Dicarbonsäure der allgemeinen Formel (III) 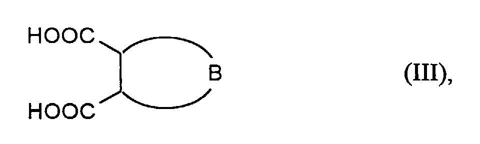 oder deren Anhydride in einem organischen Lösungsmittel, dadurch gekennzeichnet, daß die Umsetzung in Gegenwart eines aliphatischen oder aromatischen Alkohols erfolgt, wobei dieser sowohl von dem verwendeten Lösungsmittel als auch von den Verbindungen der Formeln (I) bis (III) verschieden ist.

## Beschreibung

Die vorliegende Erfindung betrifft ein Verfahren zur Herstellung von Chinophthalonen sowie die Verwendung der auf diese Weise hergestellten Chinophthalone zum Massefärben von Kunststoffen.

Chinophthalone der Formel (I) 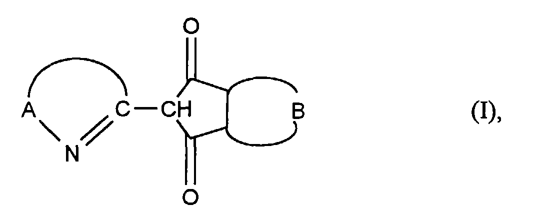 worin die Gruppierung A einen unsubstituierten oder substituierten heteroaromatischen Ring vervollständigt und die Gruppierung B einen aromatischen Ring vervollständigt, der unsubstituiert oder substituiert sein kann, sind bereits bekannt und werden in der Regel durch Umsetzung einer heterocyclischen Verbindung der allgemeinen Formel (II) 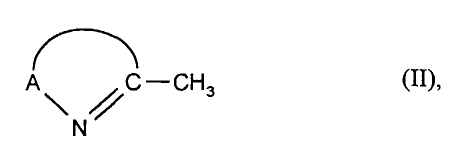 mit einer aromatischen Dicarbonsäure der allgemeinen Formel (III) 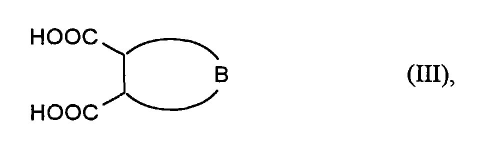 oder deren Anhydride hergestellt.

Die Umsetzung erfolgt dabei beispielsweise in der Schmelze (DE-A 1 229 663) oder in einem inerten Lösungsmittel.

Die alleinige Verwendung inerter Lösungsmittel, wie beispielsweise Nitrobenzol, o-Dichlorbenzol, Trichlorbenzol, Dimethylformamid oder Diphenylether ist beispielsweise aus GB-A 1 225 336 oder GB-A 1 199 098 bekannt und erfordert sehr hohe Temperaturen. Nachteilig bei diesem Verfahren sind die hohen Reaktionstemperaturen, die einerseits einen hohen Energieeintrag erforderlich machen und andererseits entsprechende Anteile von Nebenprodukten zur Folge haben, so daß ein erhöhter Reinigungsbedarf der erhaltenen Produkte besteht. Damit einher gehen unzureichende Raum-Zeit-Ausbeuten

Aufgabe der vorliegenden Erfindung war es ein Verfahren zur Herstellung von Chinophthalonen bereitzustellen, das die genannten Nachteile nicht mehr besitzt. Es wurde nun ein Verfahren zur Herstellung von Chinophthalonen der Formel (I) gefunden, durch Umsetzung von Verbindungen der Formel (II) mit aromatischen Dicarbonsäuren der Formel (III) oder deren Anhydriden in einem organischen Lösungsmittel, das dadurch gekennzeichnet ist, daß die Umsetzung in Gegenwart eines aliphatischen oder aromatischen Alkohols erfolgt, wobei dieser sowohl von dem verwendeten Lösungsmittel als auch von den Verbindungen der Formel (I) bis (III) verschieden ist.

Bevorzugt werden nach dem erfindungsgemäßen Verfahren Verbindungen der Formel (I) hergestellt, die der Formel (Ia) entsprechen 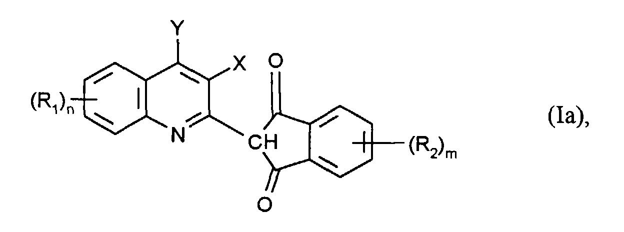 worin
- X: für H oder OH steht,
- Y: H, Halogen wie Cl, Br und F, C₁-C₄-Alkyl, C₁-C₄-Alkoxy, C₆-C₁₀-Aryl, wie Phenyl oder Carbonamid, wie CONH₂ bedeutet,
- R₁: für C₁-C₄-Alkyl, C₁-C₄-Alkoxy oder Halogen, wie Cl, Br und F steht,
- n: eine Zahl von 0 bis 4 bedeutet,
- R₂: für Halogen wie Cl, Br und F, NO₂, NH₂, NH-(C₁-C₄-alkyl), NH(acyl), OH, C₁-C₄-Alkoxy, C₁-C₄-Alkyl oder -O-Acyl, wie -O-Acetyl und -O-Propionyl steht und
- m: eine Zahl von 0 bis 4 bedeutet.

Bevorzugte heterocyclische Verbindungen der Formel (II) sind solche, die der Formel (IIa) entsprechen 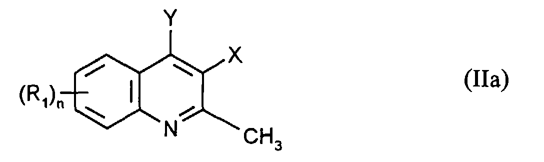 und bevorzugte aromatische Dicarbonsäuren der Formel (III) sind solche, die der Formel (IIIa) 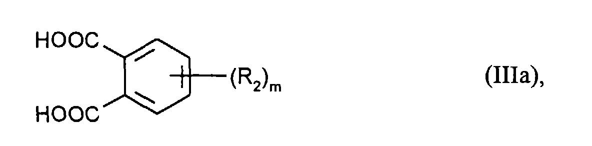 oder deren Anhydriden entsprechen, worin X, Y, R₁, R₂, n, m die obengenannte Bedeutung haben.

Bevorzugte Verbindungen der Formel (IIa) sind beispielsweise 2-Methylpyridin, 2-Methylbenzimidazol, 2-Methylbenzthiazol, 2-Methylchinazolon-(4), 8-Aminochinaldin, Chinaldin, 3-Hydroxychinaldin, 3-Hydroxy-4-carboxy-chinaldin, 3-Hydroxy-4-carbomethoxy-chinaldin oder 3-Hydroxy-6-methyl-chinaldin.

Bevorzugte Verbindungen der Formel (IIIa) oder deren Anhydride sind beispielsweise Phthalsäureanhydrid, Trimellitsäureanhydrid, Naphthalindicarbonsäureanhydrid-1,2, Tetrabromphthalsäureanhydrid, Tetrachlorphthalsäureanhydrid, 3- und 4-Nitro-phthalsäureanhydrid, 3- und 4-Aminophthalsäureanhydrid, 3- und 4-Acetylaminophthalsäureanhydrid, 4-Hydroxyphthalsäureanhydrid, Trimellitsäuremethyl- und phenylester.

Die Ausgangsverbindungen (II) und (III) bzw. (IIa) und (IIIa) sind bekannt oder nach bekannten Methoden erhältlich.

Bevorzugte Chinophthalone der Formel (I) entsprechen der Formel (Ib) 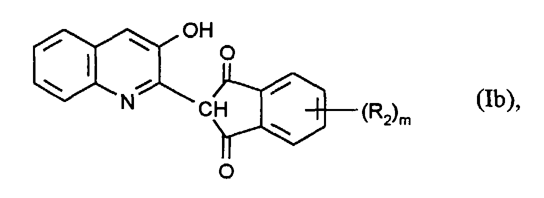 worin R₂ für Halogen, O-Acyl, NH₂ oder NH-Acyl, wie NH-Acetyl, NH-Propionyl und NH-Benzoyl steht und m für eine Zahl von 0 bis 4 steht.

Als bevorzugte organische Lösungsmittel sind zu nennen Nitrobenzol, aromatische Chlorkohlenwasserstoffe wie o-Dichlorbenzol und Trichlorbenzol, Dimethylformamid, N-Methylpyrrolidon, aromatische Ether und Ester wie Phthalsäuredimethylester, Diphenylether und Ditolylether oder eine Mischung davon.

Besonders bevorzugt ist als Lösungsmittel Ditolylether.

Als bevorzugte von dem verwendeten Lösungsmittel und den Verbindungen (I) bis (III) verschiedene aliphatische Alkohole sind gegebenenfalls substituierte C₁-C₁₂-Alkohole zu nennen, die geradkettig oder verzweigt sein können. Als bevorzugte Substituenten sind beispielsweise zu nennen: C₁-C₄-Alkoxy, C₁-C₄-Alkoxy-C₁-C₄-alkoxy, COOH, OH oder Phenyl. Als besonders bevorzugte aliphatische Alkohole kommen beispielsweise in Frage: Methanol, Ethanol, Propanol, Butanol, Dodecylalkohol, Etheralkohole wie 2-(2-Butoxy-ethoxy)-ethanol, Hydroxycarbonsäuren wie Hydroxyessigsäure, DL-Milchsäure, DL-Äpfelsäure sowie araliphatische Alkohole wie Benzylalkohol oder Mischungen davon.

Als bevorzugte von dem verwendeten Lösungsmittel und den Verbindungen (I) bis (III) verschiedenen aromatische Alkohole kommen gegebenenfalls substituiertes oder unsubstituiertes Phenol oder Naphthole in Frage. Als bevorzugte Substituenten sind beispielsweise zu nennen: C₁-C₁₄-Alkyl, insbesondere Methyl, Ethyl, Propyl, tert.-Butyl, Nonyl und Dodecyl, C₁-C₆-Alkoxy, insbesondere Methoxy, NO₂, COOH und OH. Besonders bevorzugte aromatische Alkohole sind: Phenol, 2-, 3-, 4-Methylphenol, 2-, 3-, 4-Propylphenol, 2-, 3-, 4-tert.-Butylphenol, 2,3-, 3,4-, 2,4-, 3,5-, 2,5-, 2,6-Dimethylphenol, Nonylphenol, Dodecylphenol, α-Naphthol, β-Naphthol, C₁-C₆-Alkoxyphenole wie 4-Methoxyphenol, Nitrophenol wie p-Nitrophenol, aromatische Hydroxycarbonsäuren wie Salicylsäure, 2-, 4-, 5-Methylsalicylsäure, 5-tert.-Butylsalicylsäure, Salicylsäurealkylester, 2-Hydroxy-1-naphthalin-carbonsäure, 1-Hydroxy-2-naphthalin-carbonsäure, 2,4-Dihydroxybenzoesäure oder Mischungen davon.

Ganz besonders bevorzugt werden aromatische Alkohole im erfindungsgemäßen Verfahren verwendet, insbesondere Phenol, Salicylsäure, 2-Hydroxy-1-naphthalincarbonsäure sowie 1-Hydroxy-2-naphthalincarbonsäure.

Der Anteil des verwendeten aliphatischen bzw. aromatischen Alkohols beträgt vorzugsweise 0,5 bis 2 Mol-%, bezogen auf 1 mol der Komponente der Formel (III).

Bevorzugt wird ein Molverhältnis der Verbindung (II) zu (III), das im allgemeinen von 1:2, insbesondere von 1:1,5 bis 1:1,0, vorzugsweise von 1:1,2 bis 1:1,1 beträgt.

Bevorzugt wird das Anhydrid der Dicarbonsäure (III) eingesetzt.

Verfahrentechnisch geht man im allgemeinen so vor, daß man die Komponente (II) und (III) sowie das Lösungsmittel und den aliphatischen und/oder aromatischen Alkohol unter Abdestillieren des Kondensationswassers auf die Reaktionstemperatur erhitzt.

Das erfindungsgemäße Verfahren erfolgt im allgemeinen bei Temperaturen von 100 bis 200°C, vorzugsweise bei 135 bis 200°C, insbesondere bei 160 bis 180°C.

Zweckmäßigerweise wird die Umsetzung bei Normaldruck vorgenommen, man kann jedoch auch bei 1 bis 10 bar, vorzugsweise 1 bis 5 bar arbeiten.

Bei Verwendung von aliphatischen bzw. aromatischen Alkoholen, die einen Siedepunkt von kleiner als 160°C besitzen, kann es vorteilhaft sein, das erfindungsgemäße Verfahren unter Druck durchzuführen.

Die Aufarbeitung des Reaktionsgemisches zur Isolierung der Verfahrensprodukte erfolgt in an sich bekannter Weise, und zwar vorzugsweise nach Verdünnen mit Lösungsmitteln, die mit Wasser mischbar sind, wie z.B. Methanol und Ethanol, bei Temperaturen unterhalb von 100°C, sowie anschließender Filtration bei Raumtemperatur oder erhöhter Temperatur, besonders bevorzugt durch direkte Filtration der Lösung bei einer Temperatur von 50 bis 110°C.

Die Isolierung der Chinophthalone der Formel (I) nimmt man vorzugsweise wie üblich vor, indem das Filtergut mit dem Lösungsmittel oder Wasser gewaschen und gegebenenfalls getrocknet wird.

Die nach dem erfindungsgemäßen Verfahren hergestellten Chinophthalone werden beispielsweise als Farbstoffe für Druckfarben sowie für die Spinn- und Massefärbung thermoplastischer Kunststoffe wie Polystyrol, Polyvinylchlorid, Polyamiden, Polyestern, Polyacrylnitril, Cellulosetriacetat und Celluloseacetat verwendet.

Das erfindungsgemäße Verfahren zeichnet sich gegenüber dem des Standes der Technik durch eine verfahrenstechnisch einfachere Durchführung, gute Ausbeute sowie hohe Produktreinheit aus.

Unter Massefärben von Kunststoffen werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielweise Vinylpolymere, Polyester, Polyamide oder Polycarbonate.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a.

Weiterhin geeignete Polyester sind: Polyethylenterephthalate, Polycarbonate und Celluloseester.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die nach dem erfindungsgemäßen Verfahren hergestellten Chinophthalone werden in feinverteilter Form zur Anwendung gebracht, wobei Dispergiermittel mitverwendet werden können aber nicht müssen.

Werden die nach dem erfindungsgemäßen Verfahren erhaltenen Verbindungen der Formel (I) nach der Polymerisation eingesetzt, so werden sie vorzugsweise mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguß-Verfahren zu Formteilen weiterverarbeitet.

Die Verbindungen der Formel (I) werden vorzugsweise zum Färben der genannten Polymeren in Mengen von 0,0001 bis 1 Gew..-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

### Beispiel 1

In 150 ml Ditolylether (Isomerengemisch) wurden 13,8 g Salicylsäure (0,1 mol), 25,5 g Hydroxychinaldincarbonsäure (0,126 mol) der Formel 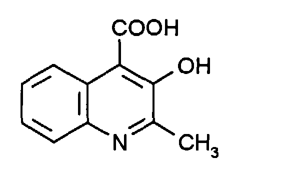 und 29,6 g Phthalsäureanhydrid (0,2 mol) eingetragen. Dann wurde unter Überleiten von Stickstoff auf 180°C erhitzt. Es wurde Kohlendioxid abgespalten und Reaktionswasser aus der Kondensationsreaktion abdestilliert. Nach ca. 12 Stunden war die Farbstoffbildung beendet. Es wurde auf 120°C abgekühlt und zur Vervollständigung der Farbstoffkristallisation wurden 250 ml Methanol zulaufen gelassen. Nach Abkühlen auf Raumtemperatur wurde der Farbstoff abgesaugt und erst mit 250 ml Methanol und anschließend mit 500 ml heißem Wasser gewaschen und im Vakuum getrocknet. Ausbeute: 34 g = 93 % der Theorie des Chinophthalons der Formel

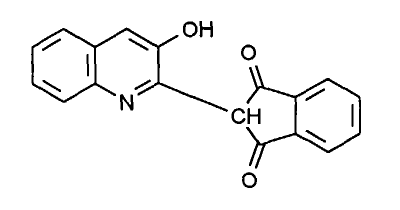

### Beispiel 2

9,5 g Phenol (0,1 mol), 25,5 g Hydroxychinaldincarbonsäure (0,126 mol) der Formel 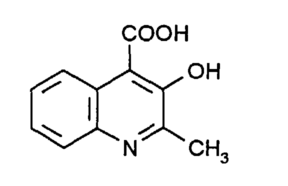 und 29,6 g Phthalsäureanhydrid (0,2 mol) wurden unter Rühren in 150 ml Ditolylether (Isomerengemisch) eingetragen. Während man über das Reaktionsgemisch einen Stickstoffstrom leitete, wurde auf 165°C erwärmt und das Reaktionsgemisch 15 Stunden auf dieser Temperatur gehalten. Nachdem die Farbstoffbildung beendet war, wurde auf 120°C abgekühlt. Danach wurde 250 ml Methanol zugegeben. Nach Abkühlen auf Raumtemperatur wurde der ausgefallene Farbstoff abgesaugt und mit 250 ml Methanol und danach mit 500 ml heißem Wasser gewaschen. Der Farbstoff wurde dann im Vakuum bei 70°C getrocknet. Ausbeute: 34,4 g = 95 % der Theorie des Chinophthalons der Formel

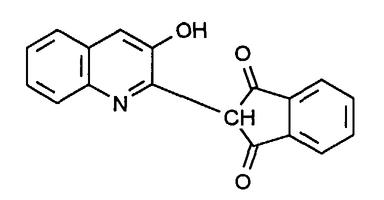

Wurde als Lösungsmitel Ditolylether durch Diphenylether oder o-Dichlorbenzol ersetzt, so erhielt man ein gleichgutes Ergebnis.

### Beispiel 3 (Vergleich)

Verfuhr man analog Beispiel 1 jedoch ohne den Zusatz des aromatischen Alkohols, so waren die Ausbeuten des Chinophthalons deutlich niedriger. Für unterschiedliche Lösungsmittel ergab sich folgendes Bild.

| | Ausbeute | |
|---|---|---|
| Lösungsmittel | g | % d. Th. |
| Ditolylether | 24 | 66 |
| Phthalsäuredimethylester | 19,5 | 53,7 |
| N-Methylpyrrolidon | 19,9 | 54,8 |

### Beispiel 4

150 ml Ditolylether (Isomerengemisch) wurden mit 26,2 g Dodecylphenol (0,1 mol), 25,5 g (0,126 mol) Hydroxychinaldincarbonsäure und 29,6 g (0,2 mol) Phthalsäureanhydrid unter Rühren gemischt und unter Stickstoff auf 180°C erwärmt. Dabei destillierte Reaktionswasser ab. Die Temperatur wurde 15 Stunden gehalten. Danach wurde auf 120°C abgekühlt und mit 250 ml Methanol versetzt. Nach dem Abkühlen auf Raumtemperatur wurde der auskristallisierte Chinophthalonfarbstoff abfiltriert, mit 250 ml Methanol gewaschen und danach mit 500 ml heißem Wasser gewaschen. Das Produkt wurde bei 70°C im Vakuum getrocknet. Ausbeute: 33,1 g = 90,5 % der Theorie.

### Beispiel 5

150 ml Ditolylether (Isomerengemisch), 15 g 4-tert.-Butylphenol (0,1 mol), 25,5 g Hydroxychinaldincarbonsäure (0,126 mol) und 29,6 g Phthalsäureanhydrid (0,2 mol) wurden unter Rühren auf 175°C erwärmt, wobei ein Stickstoffstrom übergeleitet wurde und Reaktionswasser abdestillierte. Nach 15 Stunden Reaktionszeit war die Farbstoffbildung beendet. Das Reaktionsgemisch wurde auf 120°C abgekühlt und mit 250 ml Methanol versetzte. Man rührte noch 1 Stunde bei 65 bis 68°C und ließ dann auf Raumtemperatur abkühlen. Der auskristallisierte Chinophthalonfarbstoff wurde abgesaugt und mit 250 ml Methanol gewaschen. Danach wurde mit 500 ml heißem Wasser gewaschen und der Farbstoff bei 70°C im Vakuum getrocknet. Ausbeute: 33,3 g = 91,1 % der Theorie.

### Beispiel 6

150 ml Ditolylether (Isomerengemisch), 18,8 g 2-Naphthol-1-carbonsäure (0,1 mol), 25,5 g Hydroxychinaldincarbonsäure (0,126 mol) und 29,6 g Phthalsäureanhydrid (0,2 mol) wurden unter Rühren und Stickstoffüberleiten auf 200°C erhitzt. Dabei destillierte Reaktionswasser ab. Nach 10 Stunden Reaktionszeit war das gesamte Ausgangsmaterial zum Chinophthalonfarbstoff umgesetzt. Man kühlte auf 120°C ab, fügte 250 ml Methanol zu und ließ auf Raumtemperatur abkühlen. Dann wurde abfiltriert, der Farbstoff erst mit 250 ml Methanol und dann mit 500 ml heißem Wasser gewaschen und im Vakuum bei 70°C getrocknet. Ausbeute: 33,5 g = 91,6 % der Theorie.

### Beispiel 7

150 ml Diphenylether, 11,7 g Hydroxyessigsäure (0,15 mol), 25,5 g Hydroxychinaldincarbonsäure (0,126 mol) und 29,6 g Phthalsäureanhydrid (0,2 mol) wurden unter Rühren auf 180°C erwärmt. Dabei wurde ein Stickstoffstrom übergeleitet und Reaktionswasser abdestilliert. Nach 10 Stunden war die Reaktion beendet. Man kühlte auf 120°C ab und verdünnte mit 250 ml Methanol. Danach ließ man auf Raumtemperatur abkühlen und filtrierte den auskristallisierten Chinophthalonfarbstoff ab. Das Produkt wurde mit 250 ml Methanol, danach mit 500 ml heißem Wasser gewaschen und bei 70°C im Vakuum getrocknet. Ausbeute: 32,9 g = 90,5 % der Theorie.

### Beispiel 8

150 ml Ditolylether (Isomerengemisch), 13,8 g Salicylsäure (0,1 mol), 25,5 g Hydroxychinaldincarbonsäure (0,126 mol) und 40,0 g Tetrachlorphthalsäureanhydrid (0,14 mol) wurden unter Rühren und Stickstoffüberleiten auf 170°C erhitzt. Dabei destillierte Reaktionswasser ab. Nach 12 Stunden Reaktionszeit wurde auf 120°C abgekühlt und das Reaktionsgemisch mit 250 ml Methanol verdünnt. Der Chinophthalonfarbstoff kristallisierte dabei aus und wurde durch Filtration isoliert. Der Filterkuchen wurde mit 250 ml Methanol, danach mit 500 ml heißem Wasser gewaschen und wurde darauf im Vakuum bei 70°C getrocknet. Der Farbstoff hat die Strukturformel 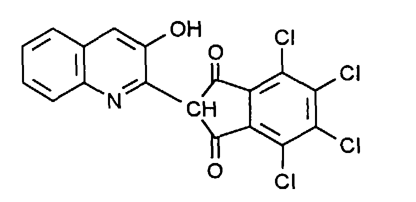 Ausbeute: 39,7 g = 93 % der Theorie.

### Beispiel 9

150 ml Ditolylether (Isomerengemisch), 8,8 g Isopentylalkohol (0,1 mol), 25,5 g Hydroxychinaldincarbonsäure (0,126 mol) und 27,4 g 4-Chlor-phthalsäureanhydrid wurden unter Rühren und Stickstoffüberleiten auf 160°C erhitzt. Dabei destillierte Reaktionswasser ab. Nach 12 Stunden Reaktionszeit wurde auf 120°C abgekühlt und das Reaktionsgemisch mit 250 ml Methanol versetzt. Der Chinophthalonfarbstoff kristallisierte aus und wurde abfiltriert. Der Filterkuchen wurde mit 250 ml Methanol, dann mit 500 ml heißem Wasser gewaschen und schließlich im Vakuum bei 70°C getrocknet. Der Farbstoff hat die Strukturformel 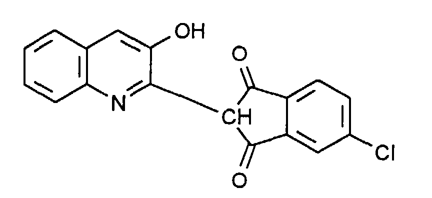 Ausbeute: 37 g = 91 % der Theorie

## Patentansprüche

1. Verfahren zur Herstellung von Chinophthalonen der Formel (I) 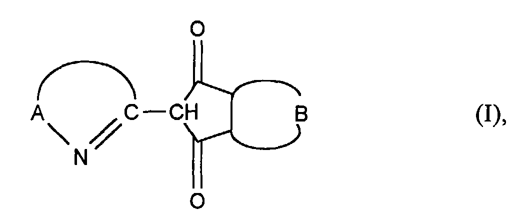 worin die Gruppierung A einen unsubstituierten oder substituierten heteroaromatischen Ring vervollständigt und die Gruppierung B einen aromatischen Ring vervollständigt, der unsubstituiert oder substituiert ist, durch Umsetzung einer heterocyclischen Verbindung der allgemeinen Formel (II) 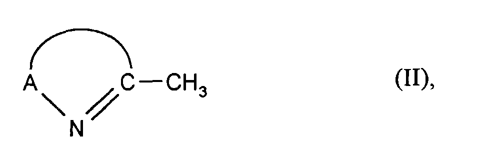 mit einer aromatischen Dicarbonsäure der allgemeinen Formel (III) 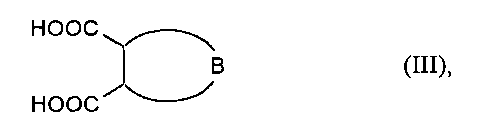 oder deren Anhydride in einem organischen Lösungsmittel, dadurch gekennzeichnet, daß die Umsetzung in Gegenwart eines aliphatischen oder aromatischen Alkohols erfolgt, wobei dieser sowohl von dem verwendeten Lösungsmittel als auch von den Verbindungen der Formeln (I) bis (III) verschieden ist.

2. Verfahren gemäß Anspruch 1, dadurch gekennzeichnet, daß Verbindungen der Formel (I) der Formel (Ia) entsprechen 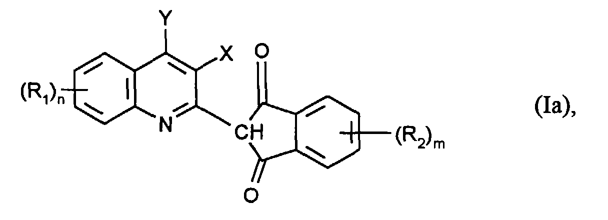 worin
X für H oder OH steht,
Y H, Halogen wie Cl, Br und F, C₁-C₄-Alkyl, C₁-C₄-Alkoxy, C₆-C₁₀-Aryl, wie Phenyl oder Carbonamid, wie CONH₂ bedeutet,
R₁ für C₁-C₄-Alkyl, C₁-C₄-Alkoxy oder Halogen, wie Cl, Br und F steht,
n eine Zahl von 0 bis 4 bedeutet,
R₂ für Halogen wie Cl, Br und F, NO₂, NH₂, NH-(C₁-C₄-alkyl), NH(acyl), OH, C₁-C₄-Alkoxy, C₁-C₄-Alkyl oder -O-Acyl, insbesondere -O-Acetyl und -O-Propionyl steht und
m eine Zahl von 0 bis 4 bedeutet.

3. Verfahren gemäß Anspruch 1, dadurch gekennzeichnet, daß die heterocyclischen Verbindungen der Formel (II) der Formel (IIa) entsprechen 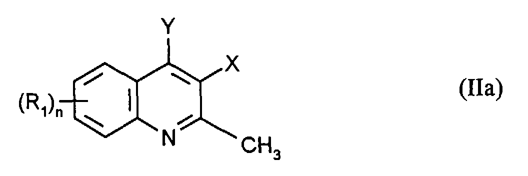 und die aromatische Dicarbonsäure der Formel (III) der Formel (IIIa) 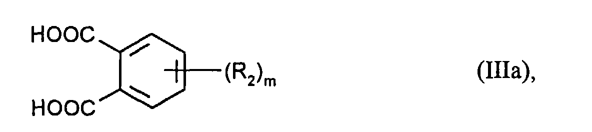 oder deren Anhydriden entsprechen, worin
X für H oder OH steht,
Y H, Halogen wie Cl, Br und F, C₁-C₄-Alkyl, C₁-C₄-Alkoxy, C₆-C₁₀-Aryl, wie Phenyl oder Carbonamid, wie CONH₂ bedeutet,
R₁ für C₁-C₄-Alkyl, C₁-C₄-Alkoxy oder Halogen, wie Cl, Br und F steht,
n eine Zahl von 0 bis 4 bedeutet,
R₂ für Halogen wie Cl, Br und F, NO₂, NH₂, NH(C₁-C₄-alkyl), NH(acyl), OH, C₁-C₄-Alkoxy, C₁-C₄-Alkyl oder -O-Acyl, insbesondere -O-Acetyl und -O-Propionyl steht und
m eine Zahl von 0 bis 4 bedeutet.

4. Verfahren gemäß Anspruch 1, dadurch gekennzeichnet, daß als Verbindung der Formel (II) 2-Methylpyridin, 2-Methylbenzimidazol, 2-Methylbenzthiazol, 2-Methylchinazolon-(4), 8-Aminochinaldin, Chinaldin, 3-Hydroxychinaldin, 3-Hydroxy-4-carboxy-chinaldin, 3-Hydroxy-4-carbomethoxychinaldin oder 3-Hydroxy-6-methyl-chinaldin eingesetzt wird.

5. Verfahren gemäß Anspruch 1, dadurch gekennzeichnet, daß als Verbindung der Formel (III) oder deren Anhydrid Phthalsäureanhydrid, Trimellitsäureanhydrid, Naphthalindicarbonsäureanhydrid-1,2, Tetrabromphthalsäureanhydrid, Tetrachlorphthalsäureanhydrid, 3- und 4-Nitro-phthalsäureanhydrid, 3- und 4-Amino-4-hydroxyphthalsäureanhydrid oder Trimellitsäuremethyl- und -phenylester eingesetzt wird.

6. Verfahren gemäß Anspruch 1, dadurch gekennzeichnet, daß die Chinophthalone der Formel (I) der Formel (Ib) entsprechen 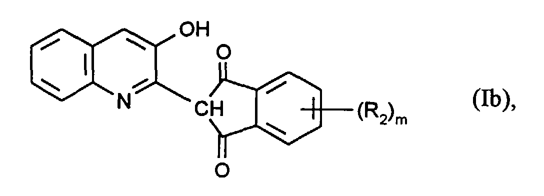 worin R₂ für Halogen, O-Acyl, NH₂ oder NH-Acyl, insbesondere NH-Acetyl, NH-Propionyl und NH-Benzoyl steht und m für eine Zahl von 0 bis 4 steht.

7. Verfahren gemäß Anspruch 1, dadurch gekennzeichnet, daß als organisches Lösungsmittel Nitrobenzol, aromatische Chlorkohlenwasserstoffe, insbesondere o-Dichlorbenzol oder Trichlorbenzol, Dimethylformamid, Methylpyrrolidon, aromatische Ether und Ester, insbesondere Phthalsäuredimethylester, Diphenylether und Ditolylether oder eine Mischung davon eingesetzt wird.

8. Verfahren gemäß Anspruch 1, dadurch gekennzeichnet, daß als von dem verwendeten Lösungsmittel und den Verbindungen (I) bis (III) verschiedene aliphatische Alkohole gegebenenfalls substituierte C₁-C₁₂-Alkohole, die gegebenenfalls geradkettig oder verzweigt sind und/oder als aromatische Alkohole gegebenenfalls substituiertes oder unsubstituiertes Phenole oder Naphthole eingesetzt werden.

9. Verfahren gemäß Anspruch 1, dadurch gekennzeichnet, daß als von dem verwendeten Lösungsmittel und den Verbindungen (I) bis (III) verschiedene aromatische Alkohole Phenol, 2-, 3-, 4-Methylphenol, 2-, 3-, 4-Propylphenol, 2-, 3-, 4-tert.-Butylphenol, 2,3-, 3,4-, 2,4-, 3,5-, 2,5-, 2,6-Dimethylphenol, Nonylphenol, Dodecylphenol, α-Naphthol, β-Naphthol, C₁-C₆-Alkoxyphenole wie 4-Methoxyphenol, Nitrophenole wie p-Nitrophenol, aromatische Hydroxycarbonsäuren wie Salicylsäure, 2-, 4-, 5-Methylsalicylsäure, 5-tert.-Butylsalicylsäure, Salicylsäurealkylester, 2-Hydroxy-1-naphthalin-carbonsäure, 1-Hydroxy-2-naphthalin-carbonsäure, 2,4-Dihydroxybenzoesäure oder Mischungen davon eingesetzt werden.

10. Verfahren zum Massefärben von Kunststoffen, dadurch gekennzeichnet, daß die nach dem Verfahren gemäß Anspruch 1 erhaltenen Chinophthalone in die geschmolzene Kunststoffmasse eingearbeitet oder den Ausgangskomponenten zur Herstellung des Kunststoffes vor der Polymerisation, zugesetzt werden.
